Europäisches Patentamt

European Patent Office

Office européen des brevets

(19)

(11) Publication number: **0 413 052 A1**

# EUROPEAN PATENT APPLICATION

(12)

(21) Application number: **89115279.5**

(22) Date of filing: **18.08.89**

(51) Int. Cl.⁵: **A61K 35/84**

(43) Date of publication of application:
**20.02.91 Bulletin 91/08**

(84) Designated Contracting States:
**AT BE CH DE ES FR GB GR IT LI LU NL SE**

(71) Applicant: **NIPPON HYPOX LABORATORIES
INCORPORATED
1759, Matsugaya Hachioji-shi
Tokyo(JP)**

(72) Inventor: **Satoh, Toshio
57-3, Nagao Jyoroku-cho
Tokushima-shi Tokushima-ken(JP)**
Inventor: **Niiro, Yasunori No. 403, 2nd
Masuoka Bldg.
1-3, Minamisako-hachiban-cho
Tokushima-shi Tokushima-ken(JP)**
Inventor: **Kakegawa, Hisao
No. 307, City-Corpo. Kimura 3-59,
Okihama-higashi
Tokushima-shi Tokushima-ken(JP)**
Inventor: **Ishii, Hiroshi
102, Aza-Minamihiraki Higashi-Umazume
Oasa-cho Naruto-shi Tokushima-ken(JP)**

(74) Representative: **Hansen, Bernd, Dr.rer.nat. et
al
Hoffmann, Eitle & Partner Patentanwälte
Arabellastrasse 4 Postfach 81 04 20
D-8000 München 81(DE)**

(54) **Process for producing Auricularia polytricha extract.**

(57) An Auricularia polytricha extract having an anti-allergic action can be obtained by extracting Auricularia polytricha with water and/or a hydrophilic organic solvent. This Auricularia polytricha extract is usable in such applications as drugs and foods.

EP 0 413 052 A1

## PROCESS FOR PRODUCING AURICULARIA POLYTRICHA EXTRACT

BACKGROUND OF THE INVENTION

(1) Field of the Invention

The present invention relates to an Auricularia polytricha extract. The Auricularia polytricha extract of the present invention has an anti-allergic action and can be used in drugs, quasi-drugs, cosmetics, foods, etc.

(2) Description of the Prior Art

In recent years, some of foods are evaluated not only by their nutrition or taste but also by their physiological activities. Researches on these efficacious foods for health are under way in many fields.

Some of mushrooms which have been used for food from of old are also used for medical purposes. Chuling and hoelen are now registered in The Pharmacopoeia of Japan as a medicine. There are literatures describing that many of edible mushrooms (fruit bodies) such as Lentinula edodes and the like contain anti-tumor polysaccharides.

However, with respect to the effect of edible mushrooms used for medicinal purposes, only very few of those mushrooms have been scientifically proven for their medical effects, except for the above cases.

SUMMARY OF THE INVENTION

The object of the present invention is to obtain a substance usable as a medicinal material or an efficacious food for health, from an edible mushroom.

The present inventors found that an Auricularia polytricha extract obtained by extracting Auricularia polytricha which has been used for food from of old, with water and/or a hydrophilic organic solvent, has an anti-allergic action.

Accordingly, the present invention lies in a process for producing an Auricularia polytricha extract having an anti-allergic action, which process comprises a step of extracting Auricularia polytricha with water and/or a hydrophilic organic solvent.

DETAILED DESCRIPTION OF THE INVENTION

The present invention is described in detail below.

In the present invention, as the solvent used for production of an Auricularia polytricha extract, there is used water, a hydrophilic organic solvent, or a mixed solvent of water and a hydrophilic organic solvent. Suitable as the hydrophilic organic solvent are solvents with low-boiling point, such as acetone, methanol, ethanol, tetrahydrofuran and the like. In the mixed solvent, the ratio of water/hydrophilic organic solvent is 1/99 (v/v) to 99/1 (v/v). As the hydrophilic organic solvent to be mixed with water, acetone is particularly preferable and is used at a water/acetone ratio of 40/60 to 60/40.

The extraction conditions such as extraction temperature, extraction time and the like have no particular restriction. However, the extraction temperature is usually -4°C to 120°C, preferably room temperature to 90°C; the extraction time is usually 10 minutes to 48 hours, preferably 2 hours to 8 hours; it is preferable to use a solvent in an amount of 1-100 parts, preferably 2-20 parts based on the dry weight of a material to be extracted, i.e. Auricularia polytricha.

After the extraction treatment, the solvent is removed from the extracted solution to obtain an Auricularia polytricha extract. The removal of the solvent is effected by means such as vacuum concentration, freeze-drying and the like. The thus obtained Auricularia polytricha extract of the present invention is a hygroscopic solid and has been confirmed to contain saccharides by the phenol-sulfuric acid method and the anthrone method and also to contain fatty acids, sterols, etc.

As is clear from Examples which are described later, the Auricularia polytricha extract of the present invention has an anti-allergic action and a very high safety and accordingly is useful for the remedy or prophylaxis of allergic diseases such as bronchial asthma, atopic dermatitis, allergic nastis and the like.

In using the extract of the present invention as a drug for the above diseases, the dose has no particular restriction and varies depending upon the type and condition of disease, age, health condition and body weight of patient, the frequency of administration, the expected effect and the type of concurrently prescribed drug, if any. However, the dose is about 0.1-10 g, preferably about 0.5-5 g per adult per day, and it is administered orally or parenterally once or more times per day.

As the dosage form in which the Auricularia polytricha extract of the present invention is administered, there can be mentioned, for example, powders, parvules, granules, tablets, capsules, suppositories, injections, lotions, ointments, etc.

Having a very high safety, the extract of the present invnetion can also be incorporated into quasi-drugs, cosmetics, foods (efficacious foods for health, health foods), etc.

The present invention is described in more detail below by way of Examples.


(1) Example of production of Auricularia polytricha extract of the present invention

100 g of a dried fruit body of Auricularia polytricha was cut into very small pieces. Thereto was added 1,000 ml of distilled water. The mixutre was heated on a water bath of 60°C for 2 hours and, when hot, filtered through a gauze. The residue was subjected to the same extraction.

The extracted solutions were combined and the insoluble materials were removed by filtration on 100 g of celite. The filtrate was concentrated under vacuum. The concentrate was freeze-dried to obtain 4.44 g of an Auricularia polytricha extract.

This extract was a yellowish white to brown hygroscopic solid. The mixture of this extract with benzene was subjected to thin-layer chromatography (silica gel 60 plate manufactured by Merck Co.; developing solvent, chloroform : methanol = 9 : 1) and then to color development by iodine. Three spots were present. These spots had Rfs of 0.83, 0.74 and 0.53 and there was tailing from the original point to around Rf = 0.06.

The same extraction was effected except that water was replaced by a water-acetone mixed solvent [50/50 (v/v)], a water-methanol mixed solvent [2/98 (v/v)] or a water-methanol mixed solvent [50/50 (v/v)], to obtain an Auricularia polytricha extract of the present invention in respective cases.


(2) Example of pharmacological test of Auricularia polytricha extracts of the present invention

There was investigated the action of the Auricularia polytricha extracts of the present invention on the reaction of histamine liberation from rat peritoneal mast cells (this reaction is widely used for the assay of an anti-allergic agent, as a model of allergic reaction taking place in vivo).

That is, in a Locke's solution were suspended about $1 \times 10^6$ (per ml of Locke's solution) of rat peritoneal mast cells. Thereto was added 2 mg/ml (final concentration) of a test sample, and the mixture was stirred to obtain a uniform solution. The solution was incubated at 37°C for 5 minutes. Then, the compound 48/80 was added and the mixutre was incubated at 37°C for 10 minutes. After ice cooling, the mixture was subjected to centrifugation at 2,500 rpm at 4°C. Both the supernatant and the residue were measured for histamine amount by the method of P. A. Shore [J. Exp. Ther., 127 , 182 (1959)] to determine the action on histamine liberation. The results are shown in Table 1. As is clear from Table 1, all of the four Auricularia polytricha extracts of the present invention showed a strong inhibition against liberation of histamine from rat peritoneal mast cells.

Table 1

| Test sample | Concentration | Inhibition for histamine liberation |
|---|---|---|
| Extract of Auricularia polytricha with water | 1 mg/ml | 44% |
| Extract of Auricularia polytricha with water-acetone (50/50) | 1 mg/ml | 94% |
| Extract of Auricularia polytricha with water-methanol (2/98) | 2 mg/ml | 44% |
| Extract of Auricularia polytricha with water-methanol (50/50) | 2 mg/ml | 37% |

EP 0 413 052 A1

(3) Example of toxicity test of Auricularia polytricha extract of the present invention

The Auricularia polytricha extracts of the present invention were tested for acute toxicity by oral administration to ddY mice. However, there was no death at a dose of 10 g/kg.

As described in detail above, there has been provided, according to the present invention, an Auricularia polytricha extract which has an anti-allergic action and a high safety and acordingly is useful as a curing or preventive agent for allergic diseases and further can be incorporated into cosmetics, foods, etc.

**Claims**

1. A process for producing an Auricularia polytricha extract having an anti-allergic action, which process comprises a step of extracting Auricularia polytricha with water and/or a hydrophilic organic solvent.

2. A process according to Claim 1, wherein the hydrophilic organic solvent is at least one member selected from the group consisting of acetone, methanol, ethanol and tetrahydrofuran.

3. A process according to Claim 2, wherein the ratio of water/the hydrophilic organic solvent is 1/99 (v/v) to 99/1 (v/v).

4. A process according to Claim 1, wherein the hydrophilic organic solvent is acetone and the ratio of water/acetone is 40/60 to 60/40.

5. A process according to Claim 1, wherein water and/or the hydrophilic organic solvent is used in an amount of 1-100 parts based on the dry weight of Auricularia polytricha.

6. A process according to Claim 5, wherein the amount of water and/or the hydrophilic organic solvent is 2-20 parts.

7. An Auricularia polytricha extract obtainable by a process according to any of the preceding claims.

8. A composition comprising as an active ingredient an Auricularia polytricha extract according to claim 7.

9. The use of the Auricularia polytricha extract according to claim 7 for the making of an anti-allergic composition.

10. The use of the Auricularia polytricha extract according to claim 7 for the making of drugs, quasi-drugs, cosmetics and foods.

4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 295 961 (KUREHA KAGAKU KOGYO K.K.) * Page 8, example 18 * | 1 | A 61 K 35/84 |
| Y | BIOLOGICAL ABSTRACTS/RMM, vol. 31, 1986, Abstract no. 31025471, Biological Abstracts Inc., Philadelphia, PA, US; L.K. SHIRAI et al.: "Effect of low dalton auricularia-polytricha extract and ionophores on the inotropic response of ciguatoxin in guinea-pig atria in-vitro", & FEDERATION PROCEEDINGS (US) 1986. Vol 45, no. 4, page 812 * Abstract * | 1 | |
| Y | NEW ENGLAND JOURNAL OF MEDICINE, vol. 304, no. 3, 1981, page 175, 4 ref, abstract no. 82620481, Washington, US; A.N. MAKHEJA et al.: " Identification of the antiplatelet substance in Chinese black tree fungus" * Whole article * | 1 | TECHNICAL FIELDS SEARCHED (Int. Cl.5) A 61 K |

The present search report has been drawn up for all claims

| Place of search | Date of completion of the search | Examiner |
|---|---|---|
| THE HAGUE | 22-12-1989 | REMPP G.L.E. |